(19) Europäisches Patentamt
European Patent Office
Office européen des brevets

(11) EP 1 029 849 A1

# (12) EUROPÄISCHE PATENTANMELDUNG

(43) Veröffentlichungstag:
**23.08.2000 Patentblatt 2000/34**

(21) Anmeldenummer: **00102140.1**

(22) Anmeldetag: **07.02.2000**

(51) Int. Cl.[7]: **C07C 235/34**, A61K 7/42, A61K 47/16, C11B 5/00, A23L 3/00

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **19.02.1999 DE 19907032**
**12.07.1999 DE 19932491**

(71) Anmelder: **HAARMANN & REIMER GMBH**
**D-37601 Holzminden (DE)**

(72) Erfinder: **Ley, Jakob-Peter, Dr.**
**37603 Holzminden (DE)**

(74) Vertreter:
**Mann, Volker, Dr. (DE) et al**
**Bayer AG**
**Konzernzentrale RP**
**Patente und Lizenzen**
**D-51368 Leverkusen (DE)**

## (54) Hydroxymandelsäureamide phenolischer Amine

(57) Hydroxymandelsäureamide phenolischer Amine können als Antioxidantien oder als Radikalfänger, insbesondere auf dem kosmetischen oder dermatologischen Gebiet, in Nahrungsmitteln sowie in kosmetischen und dermatologischen Zusammensetzungen verwendet werden.

EP 1 029 849 A1

## Beschreibung

Die Erfindung betrifft Hydroxymandelsäureamide phenolischer Amine, ein Verfahren zu deren Herstellung und ihre Verwendung als Antioxidantien oder Radikalfänger, insbesondere in kosmetischen und pharmazeutischen Zubereitungen und Nahrungsmitteln sowie zum Schutz von Zellen und Gewebe von Säugern vor den die Alterung beschleunigenden schädlichen Einflüssen von Radikalen und reaktiven Sauerstoffverbindungen. Die Erfindung betrifft ferner kosmetische und pharmazeutische Zubereitungen umfassend diese Hydroxymandelsäureamide.

Es ist erstrebenswert, Substanzen zu finden, die in physiologischen Systemen die natürlichen Abwehrmechanismen gegen freie Radikale und reaktive Sauerstoffverbindungen unterstützen oder als Schutzstoffe in Kosmetika, Pharmazeutika oder Nahrungsmitteln deren oxidationsempfindlichen Bestandteile vor Autoxidation schützen.

Antioxidantien sind Substanzen, die in im Vergleich zu dem oxidierbaren Substrat kleinen Konzentrationen die Oxidation signifikant verzögern oder gänzlich verhindern. Viele Antioxidantien füngieren gleichzeitig als Radikalfänger und/oder als Komplexbildner für Schwermetallionen.

Die Aufgabe der vorliegenden Erfindung liegt darin, neue Antioxidantien mit starker spezifischer radikalfangender und/oder antioxidativer Wirkung zu entwickeln.

Die Erfindung betrifft Hydroxymandelsäureamide phenolischer Amine der allgemeinen Formel I, deren Stereoisomere oder Gemische derselben,

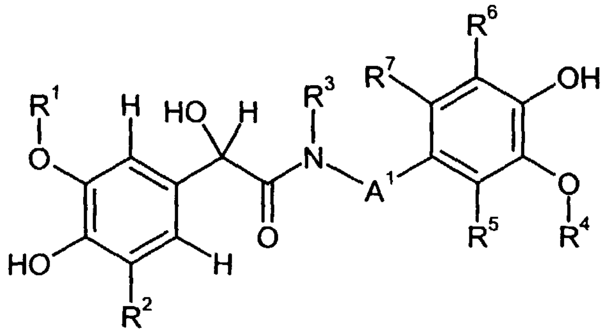

(I)

wobei

| | |
|---|---|
| $R^1$ | ein Wasserstoffatom oder ein Alkylrest mit 1 bis 4 Kohlenstoffatomen darstellt, und |
| $R^2$ | ein Wasserstoffatom oder eine Gruppe -O-$R^8$ darstellt, in der $R^8$ ein Wasserstoffatom oder ein Alkylrest mit 1 bis 4 Kohlenstoffatomen bedeutet, und |
| $R^3$ | ein Wasserstoffatom, einen Acyl-, Alkyl- oder Alkenylreste mit 1 bis 22 Kohlenstoffatomen darstellt, und |
| $A^1$ | eine Gruppe $-CH_2-$ oder eine Gruppe $-CH_2-CH_2-$ darstellt, und |
| $R^4$ | ein Wasserstoffatom oder ein Alkylrest mit 1 bis 4 Kohlenstoffatomen darstellt, und |
| $R^5$, $R^6$ und $R^7$ | unabhängig voneinander Wasserstoffatome oder Gruppen -O-$R^9$ darstellen, wobei $R^9$ ein Wasserstoffatom oder ein Alkylrest mit 1 bis 4 Kohlenstoffatomen ist. |

Bevorzugt sind Verbindungen der allgemeinen Formel I, wobei

| | |
|---|---|
| $R^1$ | ein Wasserstoffatom oder eine Methylgruppe darstellt, und |

$R^2$ ein Wasserstoffatom, eine Hydroxygruppe oder eine Gruppe $-O-CH_3$ darstellt, und

$R^3$ ein Wasserstoffatom darstellt, und

$A^1$ eine Gruppe $-CH_2-$ oder eine Gruppe $-CH_2-CH_2-$ darstellt, und

$R^4$ ein Wasserstoffatom oder eine Methylgruppe darstellt, und

$R^5$, $R^6$ und $R^7$ unabhängig voneinander Wasserstoffatome, Hydroxygruppen oder Gruppen $-O-CH_3$ darstellen.

Bevorzugt sind Verbindungen, bei denen $A^1$ eine Gruppe $-CH_2-$ darstellt.
Die insbesondere bevorzugten Verbindungen der allgemeinen Formel (I) umfassen z.B.:

N-(3 ,4-Dihydroxyphenethyl)-2-hydroxy-2-(4-hydroxy-3-methoxyphenyl)acetamid
N-(3,4-Dihydroxyphenethyl)-2-(3,4-dihydroxyphenyl)-2-hydroxyacetamid
N-(3,4-Dihydroxybenzyl)-2-hydroxy-2-(4-hydroxy-3-methoxyphenyl)acetamid
2-Hydroxy-2-(4-hydroxy-3-methoxyphenyl)-N-(3,4,5-trihydroxybenzyl)acetamid
2-(3,4-Dihydroxybenzyl)-2-hydroxy-N-(4-hydroxy-3-methoxybenzyl)acetamid
N-(3,4-Dihydroxyphenyl)-2-(3,4-dihydroxyphenyl)-2-hydroxyacetamid
2-(3,4-Dihydroxyphenyl)-2-hydroxy-N-(3,4,5-trihydroxybenzyl)acetamid

sind jedoch nicht darauf beschränkt.
Überraschenderweise wurde nun gefunden, daß die erfindungsgemäßen Hydroxymandelsäureamide phenolischer Amine besonders starke Radikalfänger und Antioxidantien sind. Insbesondere sind sie wesentlich bessere Antioxidantien und Radikalfänger als die meisten üblichen Antioxidantien.
Im Vergleich zu den aus der EP 0 900 781 bekannten Phenolsäureamiden hydroxysubstituierter Benzylamine haben die erfindungsgemäßen Hydroxymandelsäureamide phenolischer Amine eine erhöhte Wasserlöslichkeit, sodaß sie leichter in kosmetischen Zubereitungen eingesetzt werden können.
Besonders vorteilhaft als Antioxidantien oder Radikalfänger sind die Verbindungen im Sinne der Erfindung mit mehr als zwei Hydroxygruppen.
Die erfindungsgemäßen Hydroxymandelsäureamide phenolischer Amine der allgemeinen Formel 1 können mit den an sich bekannten üblichen Amidsyntheseverfahren hergestellt werden, dergestalt, daß man eine aktivierte, gegebenenfalls an den OH-Gruppen geschützten Hydroxymandelsäure mit einem gegebenenfalls an den phenolischen OH-Gruppen geschütztem phenolischen Amin oder dessen Ammoniumsalz, gegebenenfalls in Gegenwart von Lösemitteln und Hilfsbasen umsetzt. Als aktivierte Säurederivate können die Säurechloride, die Säureanhydride oder Säureester von z.B. gegebenenfalls substituierten Phenolen, N-Hydroxysuccinimid oder N-Hydroxybenzotriazol verwendet werden. Als Schutzgruppen verwendet man bevorzugt Acyl-, Carbamat- oder Ethergruppen, z.B. Acetyl-, Benzoyl-, Methoxycarbonyl-, tert.-Butoxycarbonyl-, Allyl- oder Benzylgruppen. Als Lösungsmittel können z.B. Wasser, Aceton, 1,4-Dioxan, N,N-Dimethylformamid, Tetrahydrofuran, Essigsäureethylester, Chloroform oder auch Gemische der letztgenannten Lösemittel verwendet werden. Als Hilfsbasen können z.B. Ammonium-, Alkalimetall- oder Erdalkalimetallcarbonate, -hydrogencarbonate, -hydroxide, tert. Amine und anorganische oder organische basische Ionenaustauscher verwendet werden.
Die erfindungsgemäßen Hydroxymandelsäureamide phenolischer Amine der allgemeinen Formel I werden besonders bevorzugt aus gegebenenfalls an den Hydroxygruppen mit Acetyl- oder Methoxycarbonylgruppen blockierten Hydroxymandelsäure-N-succinimidylestern mit einem phenolischen Amin oder dessen Ammoniumsalz in einem wasserhaltigen Lösungsmittelgemisch, bevorzugt einem Wasser/1,4-Dioxan-Gemisch mit einer der oben genannten Hilfsbasen bei 5 bis 100°C hergestellt. Vorteilhafterweise werden die gegebenenfalls an den Hydroxygruppen mit Acetyl- oder Methoxycarbonylgruppen blockierten Hydroxymandelsäure-N-succinimidylester aus der entsprechenden freien Säure und N-Hydroxysuccinimid mittels eines Carbodiimids, vorzugsweise N,N'-Dicyclohexylcarbodiimid, in einem aprotischen Lösungsmittel, vorzugsweise 1,4-Dioxan, Diethylether, tert.-Butylmethylether, Essigsäureethylester oder Tetrahydrofuran, bei 0 bis 50°C, vorzugsweise bei 5 bis 30°C, dargestellt, das gelöste Rohprodukt durch Filtration vom Rückstand getrennt und das Filtrat direkt im Sinne der Erfindung mit dem in Wasser vorgelegten phenolischen Amin oder dessen Ammoniumsalz und einer der oben genannten Hilfsbasen umgesetzt.
Als Hydroxymandelsäuren werden insbesondere 2-(4-Hydroxy-3-methoxyphenyl)-2-hydroxyessigsäure

(Vanillomandelsäure) und 2-(3,4-Dihydroxyphenyl)-2-hydroxyessigsäure (3,4-Dihydroxymandelsäure) sowie deren Stereoisomeren oder Gemische verwendet.

Als phenolische Amine werden insbesondere 2-(3,4-Dihydroxyphenyl)ethylamin, 3,4-Dihydroxybenzylamin, 4-Hydroxy-3-methoxybenzylamin oder 3,4,5-Trihydroxybenzylamin oder die jeweiligen Ammoniumsalze verwendet.

Die erfindungsgemäßen Hydroxymandelsäureamide können aber auch durch direkte Kondensation der freien Säuren mit den freien Aminen mit oder ohne Lösemittel erhalten werden. Als Kondensationsmittel können z.B. Carbodiimide, bevorzugt N,N'-Dicyclohexylcarbodiimid, und als Lösemittel z.B 1,4-Dioxan, Diethylether, tert.-Butylmethylether, Essigsäureethylester oder Tetrahydrofuran verwendet werden.

Die erfindungsgemäßen Hydroxymandelsäureamide phenolischer Amine erhält man aus diesen Reaktionsgemischen durch an sich bekannte Reinigungsschritte; gegebenenfalls müssen noch vorhandene Schutzgruppen mit an sich bekannten Methoden abgespalten werden.

Die erfindungsgemäßen Hydroxymandelsäureamide phenolischer Amine der allgemeinen Formel I, deren Stereoisomere sowie Gemische derselben können als Antioxidantien oder Radikalfänger zum Schutz gegen Oxidation und Photooxidation verwendet werden. Bevorzugt können sie in kosmetischen, pharmazeutischen oder dermatologischen Formulierungen oder in Nahrungsmitteln eingesetzt werden. Besonders bevorzugt werden die erfindungsgemäßen Hydroxymandelsäureamide phenolischer Amine in kosmetischen und dermatologischen Formulierungen verwendet, die wie üblich zusammengesetzt sind und zur Behandlung, zum Schutz, der Pflege und der Reinigung der Haut, der Nägel und/oder der Haare und als Schminkprodukte in der dekorativen Kosmetik dienen.

Dementsprechend betrifft die vorliegende Erfindung auch kosmetische und pharmazeutische Zusammensetzungen, insbesondere kosmetische und dermatologische Zusammensetzungen, welche die erfindungsgemäßen Hydroxymandelsäureamide phenolischer Amine in einer wirksamen Menge, nebst anderen, ansonsten üblichen Zusammensetzungsbestandteilen, umfassen. Sie enthalten 0,0001 Gew.-% bis 30 Gew.-%, bevorzugt 0,0001 bis 20 Gew.-%, insbesondere aber 0,0001 Gew.-% bis 5 Gew.-%, bezogen auf das Gesamtgewicht der Formulierung, an den erfindungsgemäßen Hydroxymandelsäureamiden phenolischer Amine der allgemeinen Formel I und können dabei als „Wasser in Öl"-, "Öl in Wasser"-, "Wasser in Öl in Wasser"-oder "Öl in Wasser in Öl"-Emulsionen, als Gele, als Lösungen z.B. in Ölen, Alkoholen oder Siliconölen, als Stifte, als Aerosole, Sprays oder auch Schäume vorliegen. Weitere übliche kosmetische Hilfs- und Zusatzstoffe können in Mengen von 5 bis 95 Gew.-%, vorzugsweise 10 bis 80 Gew.-%, bezogen auf das Gesamtgewicht der Formulierung, enthalten sein. Ferner können die Formulierungen Wasser in einer Menge bis zu 99 Gew.-%, vorzugsweise 5 bis 80 Gew.-%, bezogen auf das Gesamtgewicht der Formulierung, aufweisen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die erfindungsgemäßen kosmetischen und dermatologischen Zubereitungen können kosmetische Hilfs- und Zusatzstoffe enthalten, wie sie üblicherweise in solchen Zubereitungen vewendet werden, z.B. Konservierungsmittel, Bakterizide, Fungizide, Viruzide, Lichtfiltersubstanzen, Kühlwirkstoffe, Pflanzenextrakte, entzündungshemmende Wirkstoffe, die Wundheilung beschleunigende Stoffe, hautaufhellende Mittel, hautfärbende Mittel, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel, Silikonderivate oder Chelatbildner.

Bevorzugt können die erfindungsgemäßen Hydroxymandelsäureamide phenolischer Amine sowohl untereinander als auch mit anderen Antioxidantien kombiniert werden. Insbesondere können die erfindungsgemäßen Hydroxymandelsäureamide phenolischer Amine untereinander als auch mit Tocopherolen (Vitamin E), Tocopherolderivaten, Tocotrienolen, Ascorbinsäure (Vitamin C), Ascorbinsäurederivaten, Carotinoiden, Vitamin A oder dessen Derivaten, BHT, BHA, Gallussäureestern, Flavonoiden wie z.B. Quercetin oder Myricetin, Catechinen wie z.B. Epicatechin, Epicatechingallat, Epigallocatechin oder Epigallocatechingallat, schwefelhaltigen Molekülen wie z.B. Glutathion, Cystein, Liponsäure, N-Acetylcystein, Chelatbildnern wie z.B. EDTA oder anderen üblichen Antioxidantien kombiniert werden.

Die Menge der vorgenannten beispielhaften Antioxidantien (eine oder mehrere Verbindungen), welche nicht mit den erfindungsgemäßen Hydroxymandelsäureamiden phenolischer Amine identisch sind, kann in den erfindungsgemäßen Zubereitungen 0,0001 bis 30 Gew.-%, bevorzugt 0,0001 bis 20 Gew-%, besonders bevorzugt 0,0001 bis 5 Gew.-%, bezogen auf das Gesamtgewicht der Zubereitung, betragen.

Die erfindungsgemäßen Hydroxymandelsäureamide phenolischer Amine können aber auch zusammen mit UVA- und/oder UVB-Filtersubstanzen in den erfindungsgemäßen kosmetischen oder dermatologischen Formulierungen eingesetzt werden, wobei die Gesamtmenge an Filtersubstanzen 0,1 bis 30 Gew.-%, vorzugsweise 0,5 bis 10 Gew.-%, bezogen auf das Gesamtgewicht der Zubereitungen, betragen kann, wobei man Sonnenschutzmitteln Für Haut und Haar erhält. Als UV-Filtersubstanzen können beispielsweise 3-Benzylidencampherderivate, z.B. 3 -(4-Methylbenzyliden)-dl-campher, Aminobenzoesäurederivate, z.B. 4-(N,N-Dimethylamino)benzoesäure-2-ethylhexylester oder

Methylanthranilat, 4-Methoxycinnamate, z.B. 2-Ethylhexyl-p-methoxycinnamat oder Isoamyl-p-methoxycinnamat, Benzophenone, z.B. 2-Hydroxy-4-methoxybenzophenon, sulfonierte UV-Filter, z.B. 2-Phenylbenzimidazol-5-sulfonsäure oder 1,4-Bis(benzimidazolyl)-benzol-4,4',6,6'-tetrasulfonsäure bzw. deren Natriumsalze, Salicylate, z.B. 2-Ethylhexylsalicylat, Triazine, z.B. Octyltriazon, 2-Cyanopropensäurederivate, z.B. 2-Ethylhexyl-2-cyano-3,3-diphenyl-2-propenoat, Dibenzoylderivate, z.B. 4-tert-Butyl-4'-methoxydibenzoylmethan oder Pigmente, z.B. Titandioxide, Zirkondioxide, Eisenoxide, Siliciumdioxide, Manganoxide, Aluminiumoxide, Ceroxide oder Zinkoxide verwendet werden.

In einer bevorzugten Ausführungsform können die Hydroxymandelsäureamide phenolischer Amine im Sinne der Erfindung schließlich auch in Squalen oder Squalan gelöst und gegebenfalls mit den anderen Inhaltsstoffen zusammen mit flüchtigen oder nichtflüchtigen Siliconverbindungen als wasserfreie oder fast wasserfreie Systeme formuliert werden.

Als vorteilhafte Verkörperung der vorliegenden Erfindung wird die Verwendung erfindungsgemäßer Hydroxymandelsäureamide phenolischer Amine zum Schutz von Geweben und Zellen von Säugern, insbesondere der Haut und/oder der Haare, vor oxidativer Beanspruchung und dem schädlichen Einfluß von Radikalen angesehen.

Ebenso umfaßt die vorliegende Erfindung auch ein Verfahren zum Schutze kosmetischer oder dermatologischer Zubereitungen gegen Oxidation oder Photooxidation, wobei es sich bei diesen Zubereitungen z.B. um Zubereitungen zur Behandlung, zum Schutz und zur Pflege der Haut, der Nägel oder der Haare oder ferner auch Schminkprodukte handeln kann, deren Bestandteile Stabilitätsprobleme aufgrund von Oxidation bzw. Photooxidation bei der Lagerung mit sich bringen, dadurch gekennzeichnet, daß die kosmetischen oder dermatologischen Zubereitungen einen wirksamen Gehalt an erfindungsgemäßen Hydroxymandelsäureamiden phenolischer Amine aufweisen.

Die erfindungsgemäßen Hydroxymandelsäureamide phenolischer Amine können somit auch verwendet werden zur Herstellung pharmazeutischer, insbesondere dermatologischer Zusammensetzungen zum Schutz von Zellen und Geweben von Säugern, insbesondere des Menschen, gegenüber dem schädlichen Einfluß von freien Radikalen und reaktiven Sauerstoffspezies.

Die Menge an erfindungsgemäßen Hydroxymandelsäureamiden phenolischer Amine in diesen Zubereitungen beträgt 0,0001 Gew.-% bis 30 Gew.-%, bevorzugt 0,0001 bis 20 Gew.-%, besonders bevorzugt 0,0001 Gew.-% bis 5 Gew.-%, bezogen auf das Gesamtgewicht der Zubereitungen.

Die vorliegende Erfindung umfaßt auch die Verwendung der erfindungsgemäßen Hydroxymandelsäureamide phenolischer Amine zum Schutz von Nahrungsmitteln, bevorzugt Fette oder Fettderivate wie zum Beispiel Fettsäuren oder Fettalkohole enthaltenden Nahrungsmitteln, insbesondere aber Nahrungsmitteln, die Fette oder Fettderivate mit oxidierbaren Doppelbindungen enthalten, vor oxidativer Beanspruchung und vor dem schädigenden Einfluß von Radikalen.

Vorzugsweise beträgt die Menge an erfindungsgemäßen Hydroxymandelsäureamiden phenolischer Amine in Nahrungsmitteln 0,0001 Gew.-% bis 30 Gew.-%, besonders bevorzugt 0,0001 bis 20 Gew.-%, insbesondere aber 0,0001 Gew.-% bis 5 Gew.-%, bezogen auf das Gesamtgewicht des Nahrungsmittels.

Bevorzugt können die erfindungsgemäßen Hydroxymandelsäureamide phenolischer Amine für die erfindungsgemäße Verwendung zum Schutz von Nahrungsmitteln untereinander als auch mit anderen Antioxidantien, wie oben beispielhaft aufgeführt, kombiniert werden.

Die Menge der obengenannten beispielhaften Antioxidantien (eine oder mehrere Verbindungen), welche nicht mit erfindungsgemäßen Hydroxymandelsäureamide phenolischer Amine identisch sind, beträgt in den Nahrungsmitteln vorzugsweise 0,0001 bis 30 Gew.-%, besonders bevorzugt 0,0001 bis 20 Gew-%, insbesondere 0,0001 bis 5 Gew.-%, bezogen auf das Gesamtgewicht der Nahrungsmittel.

## Beispiele

Die nachfolgenden Beispiele sollen die vorliegende Erfindung verdeutlichen, ohne sie einzuschränken.

1. N-(3,4-Dihydroxyphenethyl)-2-hydroxy-2-(4-hydroxy-3-methoxyphenyl)acetamid (1)

2-(4-Hydroxy-3-methoxyphenyl)-2-hydroxyessigsäure (2 g, 10,1 mmol) und N-Hydroxysuccinimid (1,16 g, 10,1 mmol) wurden unter Stickstoff in 1,4-Dioxan (120 ml) gelöst, die Mischung bei Raumtemperatur mit N,N‘ -Dicyclohexylcarbodiimid (2,08 g, 10,1 mmol) versetzt und 48 h bei dieser Temperatur gerührt. Das ausgefallene Nebenprodukt wurde abfiltriert und das Filtrat im Vakuum eingedampft. Das Produkt wurde an Kieselgel mit dem Eluenten Essigsäureethylester chromatographiert. Ausbeute 1,72 g (58 %). Zu einer Lösung des Produkts (500 mg, 1,69 mmol) in 30 ml 1,4-Dioxan wurden unter Stickstoff eine Lösung von 2-(3,4-Dihydroxyphenyl)ethylaminhydrochlorid (385 mg, 2,03 mmol) in Wasser (30 ml) und anschließend Natriumhydrogencarbonat (142 mg, 1,69 mmol) hinzugefügt. Die Mischung wurde bei 50°C 4 h gerührt, anschließend mit 10 %iger Salzsäure sauer gestellt und die Reaktionslösung 3mal mit Essigsäureethylester (insgesamt 90 ml) extrahiert. Die organische Phase wurde mit gesättigter NaCl-Lösung gewaschen, über $Na_2SO_4$ getrocknet, filtriert und das Filtrat im Vakuum eingedampft. Die Reinigung erfolgte chromatographisch an Kieselgel mit dem Eluenten Essigsäureethylester. Ausbeute 231 mg eines amorphen, farblosen Festkörpers (41 % d. Th.). $^{1}$H-NMR (400 MHz, $CD_3OD$ mit Wasserunterdrückung): δ = 6,94 (1H, d 1,9 Hz), 6,79 (1H, ddd, 8 Hz, 2 Hz, 0,5 Hz), 6,74 (1H), d, 8,1 Hz), 6,654 (1H, d, 7,9 Hz), 6,647 (1H, d, 2 Hz), 6,48 (1H), dd, 8,1 Hz, 1,9 Hz), 4,87 (teilw. unterdrückt, s), 3,82 (3H, s), 3,46-3,36 (2H, m), 2,68-2,63 (2H, m) ppm; $^{13}$C-NMR (100 MHz, $CD_3OD$): δ = 175,7 (C), 149,0 (C), 133,1 (C), 131,8 (C), 121,0 (2×CH), 116,8 (CH), 116,4 (CH), 116,1 (CH), 111,6 (CH), 75,5 (CH), 56, 4 ($CH_3$), 41,8 ($CH_2$), 35,9 ($CH_2$) ppm; MS (CI-): *m/z* = 332,1 (100%, $[M-H]^-$), 664,6 (17%, $[2M-H]^-$).

2. N-(3,4-Dihydroxyphenethyl)-2-(3,4-dihydroxyphenyl)-2-hydroxyacetamid (2)

2-(3,4-Dihydroxyphenyl)-2-hydroxyessigsäure (300 mg, 1,63 mmol) und N-Hydroxysuccinimid (188 mg, 1,63 mmol) wurden unter Stickstoff in 1,4-Dioxan (20 ml) gelöst, die Mischung bei Raumtemperatur mit N,N'-Dicyclohexylcarbodiimid (336 mg, 1,63 mmol) versetzt und 16 h bei dieser Temperatur gerührt. Das ausgefallene Nebenprodukt wurde abfiltriert und das Filtrat zu einer Lösung von 2-(3,4-Dihydroxyphenyl)ethylaminhydrochlorid (309 mg, 1,63 mmol) in Wasser (20 ml) gegeben. Es wurde noch Natriumhydrogencarbonat (151 mg, 1,8 mmol) hinzugefügt und die Reaktionsmischung unter Stickstoff 1,5 h bei 50°C gerührt. Die Mischung wurde mit 5 %iger Salzsäure sauer gestellt und 3mal mit Essigsäureethylester (insgesamt 90 ml) extrahiert. Die organische Phase wurde mit gesättigter NaCl-Lösung gewaschen, über $Na_2SO_4$ getrocknet, filtriert und das Filtrat im Vakuum eingedampft. Die Reinigung erfolgte chromatographisch an Kieselgel mit dem Eluenten Essigsäureethylester. Ausbeute 266 mg eines farblosen Sirups (51 % d. Th. bez. auf die eingesetzte Säure). $^{1}$H-NMR (400 MHz, $CD_3OD$ mit Wasserunterdrückung): δ=6,83 (1H, d, 2 Hz), 6,72 (1H, d, 8 Hz), 6,674 (1H, ddd, 8 Hz, 2 Hz, 0,5 Hz), 6,672 (1H, d, 8 Hz), 6,65 (1H, d, 2 Hz), 6,50 (1H, dd, 8 Hz, 2,1 Hz), 3,43-3,35 (2H, m), 2,65 (t, 7,6 Hz) ppm; $^{13}$C-NMR (100 MHz, $CD_3OD$): δ = 175,8 (C), 146,5 (C), 146,3 (C), 146,26 (C), 144,9 (C), 133,3 (C), 131,9 (C), 121,2 (CH), 119,9 (CH), 116,9 (CH), 116,5 (CH), 116,2 (CH), 115,4 (CH), 75,4 (CH), 41,9 ($CH_2$), 36,0 ($CH_2$) ppm; MS (CI-): *m/z* = 318,8 (100 %, $[M-H]^-$), 636,4 (23 %, $[2M-H]^-$).

In analoger Weise wurden die folgenden Verbindungen in Form von farblosen oder schwach gelblichen amorphen Festkörpern erhalten:

3. N-(3,4-Dihydroxybenzyl)-2-hydroxy-2-(4-hydroxy-3-methoxyphenyl)acetamid (3)

$^{1}$H-NMR (400 MHz, $CD_3OD$ mit Wasserunterdrückung): δ = 6,94 (1H, d, 2,1 Hz), 6,87 (1H, ddd, 8,1 Hz, 2,0 Hz, 0,5 Hz), 6,75 (1H, d, 8,1 Hz), 6,73 (1H, d, 2,0 Hz), 6,68 (1H, d, 8,0 Hz), 6,60 (1H, ddd, 8,0 Hz, 2,1 Hz, 0,6 Hz), 4,94 (teilw. unterdrückt, s), 4,32 (1H, d, 14,5 Hz), 4,21 (1H, d, 14,5 Hz), 3,78 (3H, s) ppm; $^{13}$C-NMR (100 MHz, $CD_3OD$): δ = 175,5 (C), 149,0 (C), 147,7 (C), 146,4 (C), 145,7 (C), 133,3 (C), 131,6 (C), 121,3 (CH), 120,3 (CH), 116,3 (CH), 116,1 (CH), 115,9 (CH), 111,4 (CH), 75,5 (CH), 56,4 ($CH_3$), 43,5 ($CH_2$) ppm; MS (CI-): *m/z* = 318,1 (100 %, $[M-H]^-$).

4. 2-Hydroxy-2-(4-hydroxy-3-methoxyphenyl)-N-(3,4,5-trihydroxybenzyl)acetamid (4)

$^{1}$H-NMR (400 MHz, $CD_3OD$ mit Wasserunterdrückung): δ = 6,93 (1H, d, 2,1 Hz), 6,86 (1H, ddd, 8,1 Hz, 2,1 Hz, 0,5 Hz), 6,75 (1H, d, 8,1 Hz), 6,31 (2H, t, 0,6 Hz), 4,93 (teilw. unterdrückt, s), 4,27 (1H, d, 14,3 Hz), 4,14 (1H, d, 14,3 Hz), 3,79 (3H, s) ppm; $^{13}$C-NMR (100 MHz, $CD_3OD$): δ = 175,5 (C), 149,0 (C), 147,7 (C), 147,1 (2×C), 133,3 (C), 130,9 (C), 121,3 (CH), 116,0 (CH), 111,4 (CH), 107,9 (2×CH), 75,5(CH), 56,4 ($CH_3$), 43,7 ($CH_2$) ppm; MS (CI-): *m/z* = 334,0 (100 %, $[M-H]^-$), 668,7 (5 %, $[2M-H]^-$).

5. 2-(3,4-Dihydroxyphenyl)-2-hydroxy-N-(4-hydroxy-3-methoxybenzyl)acetamid (5)

$^{1}$H-NMR (400 MHz, $CD_3OD$ mit Wasserunterdrückung): δ = 6,90 (1H, dt, 2,1 Hz, 0,4 Hz), 6,78 (1H, ddd, 8,2 Hz, 2,1 Hz, 0,6 Hz), 6,74 (1H, d, 1,6 Hz), 6,72 (1H, d, 8,2 Hz), 6,71 (1H, dd, 8,0 Hz, 0,4 Hz), 6,68 (1H, dd, 8,0 Hz, 1,8 Hz), 4,90 (teilw. unterdrückt, s), 4,36 (1H, d, 14,7 Hz), 4,28 (1H, d, 14,7 Hz), 3,73 (3H, d, 0,4 Hz) ppm; $^{13}$C-NMR (100 MHz, $CD_3OD$): δ = 175,8 (C), 149,1 (C), 146,7 (C), 146,5 (C), 146,4 (C), 133,4 (C), 131,5 (C), 121,1 (CH), 119,7 (CH), 116,1 (CH), 116,0 (CH), 115,1 (CH), 112,0 (CH), 75,4 (CH), 56,3 ($CH_3$), 43,4 ($CH_2$) ppm; MS (CI-): *m/z* = 318,0 (100 %, $[M-H]^-$), 301,7 (22 %), 300,5 (34 %).

6. N-(3,4-Dihydroxybenzyl)-2-(3,4-dihydroxyphenyl)-2-hydroxyacetamid (6)

$^{1}$H-NMR (400 MHz, $CD_3OD$ mit Wasserunterdrückung): δ = 6,87 (1H, dt, 1,9 Hz, 0,5 Hz), 6,75 (1H, ddd, 8,1 Hz, 1,9 Hz, 0,5 Hz), 6,72 (1H, dd, 8,1 Hz, 0,5 Hz), 6,72 (1H, dm, 2,1 Hz, 0,3 Hz), 6,69 (1H, d, 8,0 Hz), 6,59 (1H, ddt, 8,0 Hz, 2,1 Hz, 0,6 Hz), 4,88 (teilw. unterdrückt, s), 4,25 (2H, s), ppm; $^{13}$C-NMR (100 MHz, $CD_3OD$): δ = 175,6 (C), 146,6 (C), 146,5 (C), 146,4 (C), 145,8 (C), 133,2 (C), 131,3 (C), 120,2 (CH), 119,8 (CH), 116,3 (CH), 116,1 (CH), 116,0 (CH), 115,3 (CH), 75,4 (CH), 43,6 ($CH_2$) ppm; MS (CI-): *m/z* = 304,1 (10 %, $[M-H]^-$), 608,7 (100 %, $[2M-H]^-$).

7. 2-(3,4-Dihydroxyphenyl)-2-hydroxy-N-(3,4,5-trihydroxybenzyl)acetamid (7)

$^1$H-NMR (400 MHz, $CD_3OD$ mit Wasserunterdrückung): δ = 6,86 (1H, dt, 2,0 Hz, 0,5 Hz), 6,75 (1H, ddd, 8,1 Hz, 2,0 Hz, 0,5 Hz), 6,72 (1H, dd, 8,1 Hz, 0,5 Hz), 6,30 (2H, t, 0,5 Hz), 4,88 (teilw. unterdrückt, s), 4,20 (2H, s) ppm; $^{13}$C-NMR (100 MHz, $CD_3OD$): δ = 175,5 (C), 147,1 (2×C), 146,5 (C), 146,3 (C), 133,3 (C), 130,7 (C), 119,9 (CH), 116,2 (CH), 115,4 (CH), 107,8 (2×CH), 75,4 (CH), 43,8 ($CH_2$) ppm; MS (CI-): *m/z* = 320,8 (100 %, $[M-H]^-$), 641,0 (100 %, $[2M-H]^-$).

8. Aktivität als Radikalfänger

Die Aktivität der Verbindungen nach den Beispielen 1 bis 7 als Radikalfänger wurde mit der herkömmlicher Radikalfänger verglichen. Dabei wurde der DPPH-(1,1-Diphenyl-2-picryl-hydrazyl)-Test zur Beseitigung von Radikalen angewendet.

DPPH wurde in Methanol zu einer Konzentration von 100 µmol/l gelöst. Eine Reihe von Verdünnungen der beispielhaften Verbindungen, Vitamin C, α-Tocopherol, butyliertes Hydroxytoluol und Ferulasäure wurden in Methanol hergestellt. Methanol diente als Kontrolle. 2500 µl der DPPH-Lösung wurden mit 500 µl einer jeden Testlösung gemischt und die Abnahme der Absorption bei 515 nm solange abgelesen, bis die Abnahme kleiner 2 % pro Stunde war. Die Aktivität der Testaubstanzen als Radikalfänger wurde nach folgender Gleichung berechnet:

$$\text{Aktiv. als Radikalfänger (\%)} = 100 - (\text{Absorption der Testverbindungen})/(\text{Absorption der Kontrolle}) \times 100.$$

Aus der Aktivität als Radikalfänger (%) in einer Reihe von Verdünnungen von Testverbindungen wurde für jede Testverbindung die effektive relative Konzentration $EC_{50}$ (bezogen auf die anfangs vorhandene Konzentration an DPPH, EC = c (Testverbindung)/c(DPPH)) einer Testverbindung berechnet, bei der das Radikal DPPH um 50 % beseitigt wurde. Die Ergebnisse sind in Tabelle 1 dargestellt:

Tabelle 1

| Testverbindung | $EC_{50}$ /(mol/mol) |
|---|---|
| Beispiel 1 | 0,131 |
| Beispiel 2 | 0,104 |
| Beispiel 3 | 0,195 |
| Beispiel 4 | 0,198 |
| Beispiel 5 | 0,199 |
| Beispiel 6 | 0,069 |
| Beispiel 7 | 0,085 |
| Vitamin C | 0,270 |
| α-Tocopherol | 0,250 |
| Ferulasäure | 0,350 |
| butyliertes Hydroxytoluol | 0,240 |

9. Aktivität als Antioxidantien

Die Aktivität der beispielhaften Verbindungen 1 bis 7 als Antioxidantien wurde mit der herkömmlicher Antioxidantien verglichen. Als Testsystem wurde die beschleunigte Autoxidation von Lipiden durch Luft mit oder ohne Antioxidans mit Hilfe der Rancimat-Apparatur angewendet (Rancimat ist ein eingetragenes Warenzeichen der Metrohm AG, Herisau, Schweiz).

Die beispielhaften Verbindungen, Vitamin C, α-Tocopherol, Ferulasäure und butyliertes Hydroxytoluol wurden in Methanol oder Aceton gelöst und von der jeweiligen Testlösung 100 µl zu einer vorbereiteten Ölprobe (Sojaöl, über Alumina Typ N gereinigt) von 3 g gegeben. In eine Kontrollprobe wurde nur Lösungsmittel gegeben. Durch die auf 100°C aufgeheizte, die Testlösung enthaltende Ölprobe wurde ein konstanter, trockener Luftstrom (20 l/h) geblasen und die flüchtigen Oxidationsprodukte (vorwiegend kurzkettige Fettsäuren wie Ameisen- oder Essigsäure) in einer Vorlage mit Wasser gesammelt. Die Leitfähigkeit dieser wäßrigen Lösung wurde kontinuierlich gemessen und dokumentiert. Die Oxidation von (ungesättigten) Fetten verläuft dabei eine Zeitlang nur sehr langsam und steigt dann plötzlich stark an. Die Zeit bis zum Anstieg wird als Induktionsperiode (IP) bezeichnet.

Nach der folgenden Gleichung wurde der antioxidative Index (AOI) erhalten:

$$AOI = IP_{(\text{mit Testlösung})}/IP_{(\text{Kontrollprobe})}$$

Die Ergebnisse sind in Tabelle 2 dargestellt:

Tabelle 2

| **Testverbindung** | **AOI mit 0,05% Testsubstanz** |
|---|---|
| Beispiel 1 | 13,0 |
| Beispiel 2 | 16,9 |
| Beispiel 3 | 9,8 |
| Beispiel 4 | 11,0 |
| Beispiel 5 | 12,6 |
| Beispiel 6 | 17,5 |
| Beispiel 7 | 16,4 |
| Vitamin C | 1,17 |
| α-Tocopherol | 5,05 |
| Ferulasäure | 1,79 |
| butyliertes Hydroxytoluol | 4,77 |

10. Löslichkeit in wäßrigen Medien

Die Löslichkeit der beispielhaften Verbindungen 1 bis 7 in wäßrigen Medien wurde mit der von verschiedenen Phenolsäureamiden aus EP 0 900 781 verglichen. Als wäßriges Medium wurde beispielhaft ein Gemisch aus 95 Gew.-Teilen Wasser und 5 Gew.-Teilen DMSO verwendet. Die zu testenden Verbindungen wurden in ein Glasgefäß eingewogen, mit einer definierten Menge Lösungsmittel versetzt und 5 min bei Raumtemperatur mit Ultraschall behandelt. Dieser Vorgang wurde solange wiederholt, bis die Verbindung vollständig gelöst war. Bei einer Löslichkeit von <0,05 Gew.-% wurde der Versuch abgebrochen.

Tabelle 3

| Testverbindung | Löslichkeit in einem Gemisch aus Wasser (95 Gew.-Teile) und DMSO (5 Gew.-Teile) |
|---|---|
| Beispiel 1 | 5 - 10 Gew.-% |
| Beispiel 2 | > 10 Gew.-% |
| Beispiel 3 | > 10 Gew.-% |
| Beispiel 4 | 0,5 bis 1 Gew.-% |
| Beispiel 5 | 0,05 bis 0,1 Gew.-% |
| Beispiel 6 | 0,1 bis 0,5 Gew.-% |
| Beispiel 7 | 0,5 bis 1 Gew.-% |
| Vergleich | |
| Beispiel 1 aus EP 0 900 781 | < 0,05 Gew.-% |
| Beispiel 5 aus EP 0 900 781 | < 0,05 Gew.-% |
| Beispiel 8 aus EP 0 900 781 | < 0,05 Gew.-% |
| Beispiel 14 aus EP 0 900 781 | < 0,05 Gew.-% |
| Beispiel 16 aus EP 0 900 781 | < 0,05 Gew.-% |

**Patentansprüche**

**1.** Hydroxymandelsäureamide phenolischer Amine der allgemeinen Formel (I), deren Stereoisomere oder Mischungen derselben,

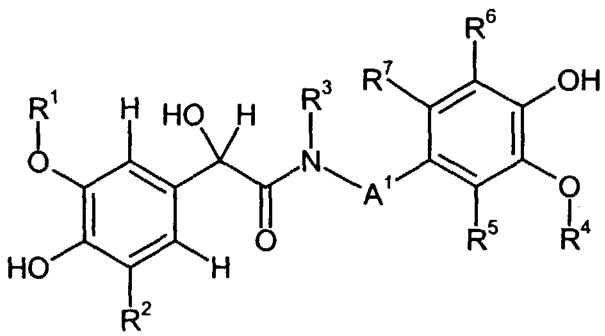

(I)

wobei

$R^1$ ein Wasserstoffatom oder ein Alkylrest mit 1 bis 4 Kohlenstoffatomen darstellt, und

$R^2$ ein Wasserstoffatom oder eine Gruppe -O-$R^8$ darstellt, in der $R^8$ ein Wasserstoffatom oder ein Alkylrest mit 1 bis 4 Kohlenstoffatomen bedeutet, und

$R^3$ ein Wasserstoffatom, einen Acyl-, Alkyl- oder Alkenylreste mit 1 bis 22 Kohlenstoffatomen darstellt, und

| | |
|---|---|
| $A^1$ | eine Gruppe $-CH_2-$ oder eine Gruppe $-CH_2-CH_2-$ darstellt, und |
| $R^4$ | ein Wasserstoffatom oder ein Alkylrest mit 1 bis 4 Kohlenstoffatomen darstellt, und |
| $R^5$, $R^6$ und $R^7$ | unabhängig voneinander Wasserstoffatome oder Gruppen $-O-R^9$ darstellen, wobei $R^9$ ein Wasserstoffatom oder ein Alkylrest mit 1 bis 4 Kohlenstoffatomen ist. |

**2.** Hydroxymandelsäureamide phenolischer Amine nach Anspruch 1, dadurch gekennzeichnet, daß sie ausgewählt sind aus der Gruppe umfassend

N-(3,4-Dihydroxyphenethyl)-2-hydroxy-2-(4-hydroxy-3-methoxyphenyl)acetamid
N-(3,4-Dihydroxyphenethyl)-2-(3,4-dihydroxyphenyl)-2-hydroxyacetamid
N-(3,4-Dihydroxybenzyl)-2-hydroxy-2-(4-hydroxy-3-methoxyphenyl)acetamid
2-Hydroxy-2-(4-hydroxy-3-methoxyphenyl)-N-(3,4,5-trihydroxybenzyl)acetamid
2-(3,4-Dihydroxyphenyl)-2-hydroxy-N-(4-hydroxy-3-methoxybenzyl)acetamid
N-(3,4-Dihydroxybenzyl)-2-(3,4-dihydroxyphenyl)-2-hydroxyacetamid
2-(3,4-Dihydroxyphenyl)-2-hydroxy-N-(3,4,5-trihydroxybenzyl)acetamid

**3.** Verfahren zur Herstellung von Hydroxymandelsäureamiden phenolischer Amine der allgemeinen Formel I, deren Stereoisomeren oder Gemischen derselben

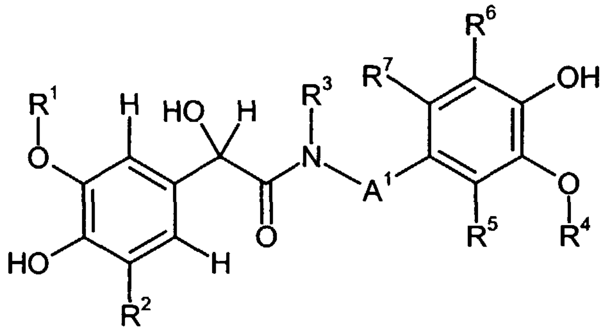

(I)

wobei

| | |
|---|---|
| $R^1$ | ein Wasserstoffatom oder ein Alkylrest mit 1 bis 4 Kohlenstoffatomen darstellt, und |
| $R^2$ | ein Wasserstoffatom oder eine Gruppe $-O-R^8$ darstellt, in der $R^8$ ein Wasserstoffatom oder ein Alkylrest mit 1 bis 4 Kohlenstoffatomen bedeutet, und |
| $R^3$ | ein Wasserstoffatom, einen Acyl-, Alkyl- oder Alkenylreste mit 1 bis 22 Kohlenstoffatomen darstellt, und |
| $A^1$ | eine Gruppe $-CH_2-$ oder eine Gruppe $-CH_2-CH_2-$ darstellt, und |
| $R^4$ | ein Wasserstoffatom oder ein Alkylrest mit 1 bis 4 Kohlenstoffatomen darstellt, und |
| $R^5$, $R^6$ und $R^7$ | unabhängig voneinander Wasserstoffatome oder Gruppen $-O-R^9$ darstellen, wobei $R^9$ ein Wasserstoffatom oder ein Alkylrest mit 1 bis 4 Kohlenstoffatomen ist, |

dergestalt, daß man eine in Form des Säurechlorids, des Säureanhydrids oder eines Säureesters von gegebenenfalls substituierten Phenolen, N-Hydroxysuccinimid oder N-Hydroxybenzotriazol aktivierte, gegebenenfalls an den phenolischen OH-Gruppen geschützte Hydroxymandelsäure mit einem gegebenenfalls an den phenolischen OH-Gruppen geschützen phenolischen Amin oder dessen Ammoniumsalz, gegebenenfalls in Gegenwart von Lösemitteln und Hilfsbasen umsetzt und die gegebenenfalls vorhandenen Schutzgruppen abspaltet.

**4.** Verfahren nach Anspruch 3, dadurch gekennzeichnet, daß man als Hydroxymandelsäuren 2-(4-Hydroxy-3-methoxyphenyl)-2-hydroxyessigsäure (Vanillomandelsäure) oder 2-(3,4-Dihydroxyphenyl)-2-hydroxyessigsäure (3,4-Dihydroxymandelsäure) sowie deren Stereoisomeren oder Gemische verwendet.

**5.** Verfahren nach Anspruch 3 und 4, dadurch gekennzeichnet, daß man als phenolische Amine 2-(3,4-Dihydroxyphenyl)ethylamin, 3,4-Dihydroxybenzylamin, 4-Hydroxy-3-methoxybenzylamin oder 3,4,5-Trihydroxybenzylamin oder die jeweiligen Ammoniumsalze verwendet.

**6.** Verwendung der Hydroxymandelsäureamide phenolischer Amine nach Anspruch 1 oder 2 zur Verwendung als Antioxidantien oder als Radikalfänger.

**7.** Kosmetische und/oder pharmazeutische Zusammensetzung enthaltend wenigstens eines der Hydroxymandelsäureamide phenolischer Amine gemäß der Ansprüche 1 und 2.

**8.** Kosmetische und/oder pharmazeutische Zusammensetzungen enthaltend 0,0001 bis 30 Gew.-% eines der Hydroxymandelsäureamide phenolischer Amine gemäß den Ansprüchen 1 und 2.

**9.** Verwendung der Hydroxymandelsäureamide phenolischer Amine nach Anspruch 1 und 2 als Antioxidantien oder Radikalfänger.

**10.** Verwendung der Hydroxymandelsäureamide phenolischer Amine nach Anspruch 9 in kosmetischen und/oder pharmazeutische Zusammensetzungen.

**11.** Verwendung der Hydroxymandelsäureamide phenolischer Amine nach Anspruch 9 in Sonnenschutzmitteln.

**12.** Verwendung der Hydroxymandelsäureamide phenolischer Amine nach Anspruch 9 in Nahrungsmitteln.

Europäisches Patentamt

# EUROPÄISCHER RECHERCHENBERICHT

Nummer der Anmeldung
EP 00 10 2140

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 5 183 828 A (VAN T RIET BARTHOLOMEUS ET AL) 2. Februar 1993 (1993-02-02)<br>* Ansprüche *<br>----- | 1 | C07C235/34<br>A61K7/42<br>A61K47/16<br>C11B5/00<br>A23L3/00 |

RECHERCHIERTE SACHGEBIETE (Int.Cl.7)
C07C
A61K
C11B
A23L

Der vorliegende Recherchenbericht wurde für alle Patentansprüche erstellt

| Recherchenort | Abschlußdatum der Recherche | Prüfer |
|---|---|---|
| DEN HAAG | 26. Mai 2000 | Pauwels, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 00 10 2140

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-05-2000

| Im Recherchenbericht angeführtes Patentdokument | Datum der Veröffentlichung | Mitglied(er) der Patentfamilie | Datum der Veröffentlichung |
|---|---|---|---|
| US 5183828 A | 02-02-1993 | US 4623659 A | 18-11-1986 |
| | | US 4942253 A | 17-07-1990 |
| | | AT 59553 T | 15-01-1991 |
| | | AU 589111 B | 05-10-1989 |
| | | AU 3013084 A | 18-12-1984 |
| | | CA 1339221 A | 05-08-1997 |
| | | DE 3483822 D | 07-02-1991 |
| | | DK 13785 A | 11-01-1985 |
| | | EP 0144396 A | 19-06-1985 |
| | | FI 843681 A | 24-11-1984 |
| | | IT 1209548 B | 30-08-1989 |
| | | JP 5078299 A | 30-03-1993 |
| | | JP 5001780 B | 11-01-1993 |
| | | JP 60501409 T | 29-08-1985 |
| | | KR 9204187 B | 30-05-1992 |
| | | NO 843739 A | 06-12-1984 |
| | | WO 8404676 A | 06-12-1984 |

EPO FORM P0461

Für nähere Einzelheiten zu diesem Anhang : siehe Amtsblatt des Europäischen Patentamts, Nr.12/82